# EUROPEAN PATENT SPECIFICATION

(11) **EP 0 496 830 B1**
(45) Date of publication and mention of the grant of the patent: **31.03.1999**
(21) Application number: 90917159.7
(22) Date of filing: 16.10.1990
(51) Int. Cl.: C07D 489/02, C07D 489/08, A61K 31/485

(54) **TOTAL SYNTHESIS OF NORTHEBAINE, NORMORPHINE, NOROXYMORPHONE ENANTIOMERS AND DERIVATIVES VIA N-NOR INTERMEDIATES**
TOTALE SYNTHESE VON NORTHEBAIN-, NORMORPHIN-, NOROXYMORPHON-ENANTIOMEREN UND -DERIVATEN VIA N-NOR-ZWISCHENVERBINDUNGEN
SYNTHESE TOTALE D'ENANTIOMERES ET DE DERIVES DE NORTHEBAINE, NORMORPHINE, NEUROXYMORPHONE PAR DES INTERMEDIAIRES N-NOR

(30) Priority: 16.10.1989 US 421900
(43) Date of publication of application: 05.08.1992
(73) Proprietor: THE UNITED STATES OF AMERICA as represented by the Secretary UNITED STATES DEPARTMENT OF COMMERCE, Washington, DC 20231 (US)
(72) Inventor: RICE, Kenner, Cralle, Bethesda, MD 20817 (US); NEWMAN, Amy, Hauck, Silver Spring, MD 20910 (US)
(74) Representative: Jump, Timothy John Simon
(86) International application number: US9005911
(87) International publication number: WO9105768

(56) References cited:
- WO-A-80/00841
- WO-A-81/00409
- CA-A- 0 913 077
- FR-A- 2 208 902
- US-A- 3 299 072
- US-A- 4 141 897
- US-A- 4 368 326
- US-A- 4 472 253
- US-A- 4 521 601
- US-A- 4 613 668
- US-A- 4 639 520
- (Wallace) 16 October 1985 see Abstract, pages 10-28.
- Chemische Berichte, Volume 57, issued 1924, SPEYER, et al., "Uber die Einwerkung von Bromcyan auf Oxy-kodeinon und Dihydro-oxykodeinon, pages 1427- 1430.
- K.W. BENTLEY, "The Chemistry of the Morphine Alkaloids", published 1954, Oxford, at the Clarendon Press, see pages 258-259, 262.
- CHEMICAL ABSTRACTS, Volume 56, issued 1962, A.C. CURRIE, et al., "14-Hydroxynor codeine and its d Derivatives.", Abstract No. 11638 b-c, g, J. Chem. Soc. 1961, 4693-700 (Eng.).
- CHEMICAL ABSTRACTS, Volume 62, issued 1965, A.C. CURRIE, et al., "Derivatives of 14-Hydroxynor-codeinone and their production.", Abstract 2801h-2802 a-c, Brit. 975,602(Cl. C07d), Nov. 18, 1964.
- Journal of the Chemical Society(C), 1966, BARTELS-KEITH, "Syntheses Related to Northebaine. Part 1. Northebaine and N-Allyl-northebaine.", pages 617-624.
- Journal of Organic Chemistry, Volume 45, No. 15, issued 18 July, 1980, KENNER C. RICE, "Synthetic Opium Alkaloids and Derivatives. A Short Total Synthesis of (+)-Dihdrothebainone, (+)-Dihydrocodein-one, and (+)- Nordihydrocodeinone as an Approach to a Practical Synthesis of Morphine, Codeine, and Cogeners., pages 3135-3137.
- CHEMICAL ABSTRACTS, Volume 101, issued 1984, K.C. RICE, "Short total synthesis of morphinan compounds which use cyclization of cycloalkylcarbonyl compounds selected from cyclopropylcarbonyl and cyclocarbonyl-butyl.", Abstract No. 55403t, Abstract of U.S. Pat. US 564,515 13 Apr. 1984, Appl. 22 Dec. 1983; 20 pp. Avail NTIS Order No. PAT-APPL-6-564 515.

## Description

The present invention relates to a total synthesis proceeding from nordihydrocodeinone (4) as in the post scheme, and a key aspect is that a facile method has now been developed, utilizing said nordihydrocodeinone intermediates throughout, for conversion of (4) to northebaine (12), noroxymorphone (19), norcodeine (39), normorphine (42) and derivatives. Since both (+)- and (-)-nordihydrocodeinone are equally available by the earlier Rice patents, the present disclosure is applicable and pertains to synthesis of compounds of both the natural (-)- and unnatural (+)-stereochemical series.

previous Rice patents, US 4,368,326 and 4,521,601, disclose the total synthesis of either enantiomer of nordihydrocodeinone (4) and the subsequent conversion of this compound to the N-methyl derivative, dihydrocodeinone (5), which is a useful intermediate in this context for synthesis of codeine (6), morphine (7) and thebaine (8); (see Chart 1, illustrated with the natural stereo-chemistry). The latter three compounds are the only raw materials obtained from opium which are of value in the production of narcotics, narcotic antagonists and the agonist-antagonist drugs. In the standard manufacturing process of the narcotic antagonists, naloxone (20), naltrexone (21), nalmefene (22), and the agonist-antagonist nalbuphine (26), (-)-thebaine [(-)-8] is obtained by extraction from opium and utilized as starting material. (-)-Codeine has also been advanced as a possible starting material for these compounds. Since both natural thebaine and codeine have a methyl substituent on the nitrogen, removal of the methyl group and replacement with a cycloalkylmethyl or allyl group and other structural alterations are necessary in order to obtain the desired pharmacological profile in the final products. For example, in the synthesis of the narcotic antagonists, natural thebaine is sequentially oxidized to 14-hydroxycodeinone, reduced to 14-hydroxydihydrocodeinone, O-demethylated to oxymorphone. The methyl group is then removed by the following sequence: acetylation to the 3,14-diacetoxy derivative, reaction with cyanogen bromide or a chloroformate ester, and hydrolyzed to noroxymorphone which is finally alkylated with the appropriate allyl halide.

A major disadvantage of the commercial process is the multistep removal of N-methyl group involving the acylation reaction, reaction with cyanogen bromide or phosgene-derived chloroformate (which are toxic and otherwise dangerous reagents), and the hydrolysis step. The hydrolysis of the N-cyano employed in the standard process requires prolonged heating with a large excess of 25% sulfuric acid that results in partial destruction of the desired noroxymorphone

Accordingly, in a first aspect of the invention, there is provided a process of producing an intermediate for the production of opiate narcotics, agonist-antagonist drugs, and stereoisomers thereof, comprising the steps of:
(a) refluxing nordihydrocodeinone having an unsubstituted N-substituent with an organic acid and trialkylorthoformate in a short chain alcohol ROH, where R is C₁-C₄ alkyl under an inert atmosphere to yield an alcoholic mixture of dialkyl ketal (I): 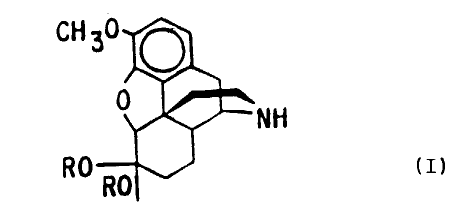 and a delta-6-enol ether (II): 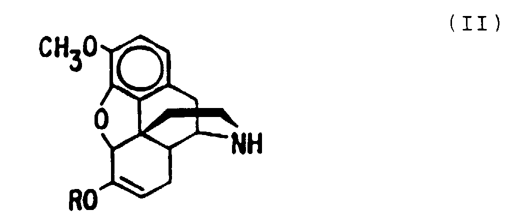
(b) completely converting the dialkyl ketal (I) in said mixture to the delta-6-enol ether (II);
(c) reacting the delta-6-enol ether (II) from step (b) with an organic acid and a halogenating agent to yield a hydrohalide salt of a 7-halo-dialkyl ketal (III) : 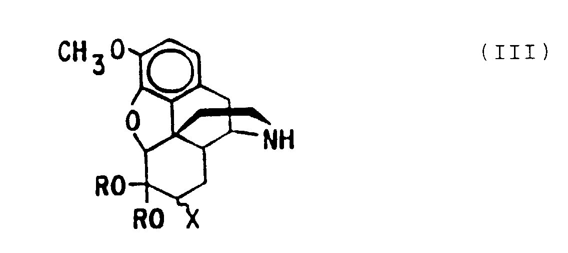 where X=halo, wherein said process occurs without substitution of said N-substituent.

In a second aspect of the invention, there is provided hydrobromide salt of optically active crystalline (+)7-bromodialkyl ketal of nordihydrocodeinone (III) and salts thereof.

Preferred embodiments of the invention in either of its aspects are as defined in the sub-claims.

The instant invention is a process in which nordihydrocodeinone (4), an early intermediate in the total synthesis of codeine, morphine and thebaine, Chart 1, is converted through intermediates without substituents on nitrogen to a number of versatile N-nor intermediates. These compounds can serve as precursors for a number of important drugs (with the natural opiate stereochemistry) currently used in the practice of medicine. The process thus eliminates the need for thebaine for the total synthesis of these drugs. Since nordihydrocodeinone is directly available by the total synthesis as either the (+)- or (-)-enantiomer, optional access is provided to either the natural or unnatural opiate series. Since the N-nor intermediates can be N-alkylated at any stage to afford a desired N-substituted product, the process is much more versatile than the classical route, and substantially shorter because the N-demethylation sequence is eliminated.

For total synthesis, the instant invention thus differs from the prior art by not utilizing thebaine, that is, it is unnecessary to introduce the methyl group (4-->5, Chart 1) and then remove it after further transformation of thebaine. This results in a shorter and more economical process by eliminating a substantial number of steps and requirements for labor and raw materials. Furthermore, each step in the process gives very high yields and each isolated intermediate is obtained pure, or very nearly so, by simple crystallization and washing.

A description of the preferred process follows (Chart 2-7). Anhydrous nordihydrocodeinone (4) as either enantiomer is treated with a mixture of trialkyl orthoformate, corresponding short chain alkyl alcohol, such as methanol and 5-sulfosalicylic acid, methane sulfonic acid or other organic acid to give a mixture of the ketal (9) and enol ether (10) Tetrahydrofuran is added and distilled to completely convert the mixture to enol ether (10) which can be isolated in 90% yield. Conversion can also be accomplished by addition of a strong base, such as potassium t-butoxide, or other organic base. Treatment of the enol ether (10) in a short chain alcohol such as methanol with halogenating reagent, preferably a brominating reagent such as N-bromoacetamide and organic acid such as methanesulfonic acid, 5-sulfosalicylic acid, give 88% yield of crystalline bromoketal (11) as the hydrobromide. Treatment of compound (11) with a strong organic base, such as potassium t-butoxide in dimethylsulfoxide or sodium hydride, sodium amide in aprotic organic solvent then gives northebaine (12) in 97% yield. Oxidation of northebaine (12) with performic acid formed in situ afforded pure 14-hydroxynorcodeinone (13) in 90% yield after crystallization from any short chain alcohol, such as methanol or other suitable purification technique. Catalytic hydrogenation of compound (13) (generally with palladium) smoothly affords crude nearly pure noroxycodone (18) in quantitative yield. Addition of formaldehyde in this hydrogenation provides the clinically used agonist percodan (17). O-Demethylation of percodan (17) by standard procedures gives numorphan (16) also used clinically as a potent narcotic agonist. Brief treatment (O-demethylation) of compound (18) with BBr₃ or other standard O-demethylation procedure then gives noroxymorphone (19). Percodan (17) and numorphan (16) are also available by N-methylation of compounds (18) and (19), respectively, using standard methods.

Noroxymorphone (19) is a centrally important precursor for naloxone (20), naltrexone (21) nalmefene (22), all valuable narcotic antagonists in the natural stereochemical series (Chart 3). N-alkylation of compound (19) with allyl or cyclopropylmethyl bromide by standard methods gives naloxone and naltrexone, respectively. These compounds can also be obtained (Chart 3) by alkylation of noroxycodone (18) to compounds (23) and (24), followed by O-demethylation with BBr₃ or other suitable reagent. Reaction of naltrexone (21) with methylene triphenylphosphorane according to standard protocol then gives nalmefene (22). Stereoselective reduction of 14-hydroxynorcodeinone (13) generally with any stereoselective reduction agent, preferably an alkali borohydride such as sodium borohydride, gives 14-hydroxynorcodeine (14) to the exclusion of the isocodeine derivatives (Chart 2). Catalytic hydrogenation of compound (14), typically with palladium, provides a quantitative yield of 14-hydroxynor-dihydrocodeine (15).

As shown in Chart 4, N-cyclobutyl-methylation of (15) to (25) followed by O-demethylation of (25) gives nalbuphine (26, Nubaine), a clinically useful agonist-antagonist drug. Alternately, O-demethylation of (15) to 14-hydroxydihydronormorphine (27) followed by N-cyclobutylation gives nalbuphine.

The potent narcotic agonist Foxy which is useful in pharmacological studies can also be obtained (Chart 5) from (-)-(14) by hydrogenation in the presence of formaldehyde to (28), previously converted to Foxy (29). Hydrogenation of (14) to (15) followed by N-methylation of (15) gives (28) the same intermediate to Foxy. The potent narcotic antagonist cyclofoxy (30), when labeled with [¹⁸F], was recently shown to be a highly useful agent for labeling opiate receptors in the living brain by positron emission tomography. This compound is easily available from (15) by N-cyclopropylation to (31) which is O-demethylated to the corresponding 6 α-naltrexol 32 and treated as previously described by prior art. Alternately, conversion of (27) to the N-cyclopropylmethyl derivative affords the same intermediate naltrexol. Previously, the naltrexol was prepared by borohydride reduction of naltrexone and required chromatographic fractionation to remove the corresponding 6-B-isomer.

An additional utility of the instant invention is that it renders N-alkylnorthebaines including thebaine (8) readily available by total synthesis in either the natural or unnatural series by simple alkylation or reductive alkylation of the appropriate enantiomer of northebaine (12) as shown in Chart 6. For example, treatment of northebaine with cyclopropylmethylbromide gives cyclopropylmethylnorthebaine (33), an intermediate useful for synthesis of buprenorphine (34), a state of the art agonist antagonist drug which is effective by sublingual administration. The potent antagonist diprenorphine (35) can also be prepared from N-cyclopropylmethylnorthebaine by prior art procedures which were demonstrated only in the natural stereochemical series.

It is thus clear that the instant invention is an extremely practical thebaine-free total synthesis of all clinically used 14-hydroxymorphinans and other (-)-thebaine derivatives, such as (34) and (35), as well as other important compounds derived from opium. The process is shorter and more flexible than total synthesis through thebaine and is also applicable to synthesis of the unnatural opiate series (not available from opium derivatives), some members of which are potent antitussives.

### Applicability of the Process for Utilization of Natural Thiamine for Semisynthetic Production of Drugs

As discussed above, the instant invention eliminates requirements for thebaine for the production of medically useful drugs by total synthesis. The process is, however, applicable to synthesis of drugs from opium derived starting material via semisynthetic northebaine since northebaine is an intermediate in the invention and existing methodology developed by others permits facile, high-yielding synthesis of (-)-northebaine from natural thebaine.

The instant invention can also be used to prepare codeine (6), morphine (7), and related compounds via norcodeine (39) and normorphine (42) in both the natural and unnatural opiate series. In this sequence (Chart 7), the norbromoketal (11) is treated with a strong base, for example an organic base such as potassium t-butoxide, or sodium hydroxide or sodium amide, in an ether or hydrocarbon solvent such as tetrahydrofuran to give norcodeinone ketal (37). Hydrolysis to norcodeinone (38), followed by reduction, e.g., with sodium borohydride or other alkali butoxide, gives norcodeine (39). O-demethylation of norcodeine with BBr₃ e.g., then gives normorphine (42). N-methylation of (39) and (42) provides codeine (6) and morphine (7), respectively, as either the natural or unnatural isomers, depending upon the absolute configuration of (11). Codeine can also be obtained from norcodeinone (38) by treatment with formaldehyde and sodium borohydride or cyanoborohydride. Codeine can also be obtained by reductive alkylation of norcodeine with formaldehyde and borohydride.

As an example of the utility of (39) and (42), nalorphine 3-O-methyl ether (40) and nalorphine (41, Nalline) can be obtained by alkylation with allyl bromide under standard conditions. Treatment of 40 with BBr₃ also gives nalorphine (41), a clinically used narcotic antagonist.

Melting points were determined on a Thomas-Hoover melting point apparatus and are corrected. Elemental analyses were performed by Atlantic Microlabs, Atlanta, Georgia. IR spectra were determined on a Beckman IR 4230 spectrophotometer; mass spectra (chemical ionization NH₃) were obtained on a Finnegan 1015 spectrometer (NH₃-CI) and l_{N}-NMR were obtained on Varian XL 300 or Varian 220 NMR spectrometers. Optical rotations were measured with a Perkin-Elmer Model 241 polarimeter. Silica gel GF plates (Analtech, Newark, Del.) were employed for thin layer chromatography (TLC) and a Hewlett Packard 5880A gas chromatograph with a 6-ft methyl silicone column was used for capillary gas chromatography (GC). Acidification of non-aqueous solutions in some cases was monitored by application of an aliquot to moist pH indicator sticks (E. Merck). TLC and GC were used to compare all enantiomeric compounds throughout the synthesis and as criteria for purity. All synthesized products had Rf values and retention times which were identical with those of their authentic (-)-enantiomers. All other spectral data obtained on each (+)-enantiomer were identical to that of authentic (-)-enantiomers with the exception of opposite optical rotation.

### Applicability of the Instant Invention to the Natural or Unnatural Opiate Series

The following examples beginning with (+)nordihydrocodeinone [(+)-4] illustrate the instant invention in the unnatural (+)-opiate series. For synthesis of the (-)-compounds with the natural opiate absolute configuration of the carbon nitrogen skeleton, it is merely necessary to employ (-)-nordihydrocodeinone [(-)-4] in an exact replication of the process. In this manner, the (-)-enantiomers described above are obtained which are identical in every respect with authentic samples prepared by classical routes from opium. Obviously, (-)-northebaine prepared by N-demethylation of natural (-)-thebaine could be utilized instead of the identical substance prepared by total synthesis.

### Example 1

### (+)-8,14-Dihydronorthebaine, [(+)-10]

A solution of 22.8 g 5-sulfosalicylic acid (90 mmol) in 8 mL MeOH was added to 38 g trimethyl orthoformate (352 mmol) in 8 mL MeOH and allowed to stir at reflux for 5 min, under an atmosphere of argon. A solution of 22.8 g (64 mmol) of anhydrous (+)-nordihydrocodeinone [(+)-4] in 30 mL MeOh was added dropwise at a constant rate to the refluxing reaction mixture via addition funnel over 1 h. If the addition is too rapid the insoluble salt of the starting material will crystallize and the reaction will not go to completion because of the low solubility of this salt. The addition funnel was rinsed with 10 mL dry THF and the reaction mixture stirred at reflux for 30 min at which time GC analysis of the basic fraction showed complete loss of starting material and a mixture of 90% ketal and 10% 8,14-dihydrothebaine. The condenser was replaced with a fractional distillation apparatus and 30 mL MeOH was removed by distillation. The reaction mixture became cloudy, as crystals (5-sulfosalicyclic acid salt of 10) separated. Dry THF was added at the rate of distillation via addition funnel; after distillation of 300 mL THF, GC (RT = 4.90, 220°C) and TLC analysis showed reaction was complete. The reaction mixture was poured, under an atmosphere of argon, into 150 mL of vigorously stirred 10% NaOH, at 0°C, and allowed to stir for 5 min; the reaction flask was washed with 30 mL THF. Removal of the residual THF in vacuo, followed by extraction with 1 x 100 mL and 2 x 50 mL CHCl₃ then washing the combined organic extract with 1 x 50 mL H₂O and removal of volatiles in vacuo resulted in a brown syrup. Crystallization from EtOAc afforded 17.09 g (90%) of (+)-10; mp 148.5-150.5°C (mp lit 152--152.5°C) which was homogeneous and identical to the (-)-enantiomer by TLC and homogenous by TLC and GC analysis (220°C). Anal. calc. for C₁₈H₂₁NO₃: C, 72.26; H, 7.02: N, 4.64. Found: C, 72.16; H, 7.12; N, 4.64. [α]_{D}²⁰ + 217.6 (c, 1.27, CHCl₃).

### Example 2

### (+)-7-Bromonordihydrocodeinone Dimethyl Ketal Hydrobromide, [(+)-11].HBr

A solution of 27.0 g (+)-10 (90 mmol) in 400 mL MeOH was mechanically stirred at 0°C under an atmosphere of argon. A solution of 9.45 mL methanesulfonic acid (126 mmol) in 75 mL MeOH was added and the reaction mixture was allowed to stir at 0°C for 10 min N-bromoacetamide (NB; 11.2 g, 81 mmol) was slowly added portionwise to the reaction mixture. After stirring for 20 min, 1.0 g NBA (6.3 mmol) was added followed by 20 min stirring and an additional 250 mg NBA (1.8 mmol). After 10 min additional stirring, the reaction was complete by GC and TLC analysis. Saturation with NH₃ gas to pH 9.5 followed by removal of MeOH, in vacuo, resulted in a yellow syrup. The mixture was treated with 200 mL of 20% NH₄OH and extracted with 3 x 100 mL of CHCl₃. The organic phase was washed with 100 mL 20% NH₄OH/H₂O and evaporated to a syrup. Distillation of toluene followed by isooctane, and drying in high vacuum gave 37.55 g of off-white foam that was 95% pure by GC analysis. This material was dissolved in 40 mL hot MeOH and acidified with freshly prepared HBr/MeOH. Cooling to 0°C gave 38.1 g of white crystalline (+)-11.HBr (88%), mp 232°C. Anal. calc. for C₁₉H₂₄No₄.HBr: C, 46.47; H, 4.09; N, 2.58. Found: C, 46.30; H, 5.18; N, 2.80. [a]_{D}²⁸ + 116.0 (c, 1.06, CHCl₃).

### Example 3

### (+)-Northebaine, [(+)-12]

To a mixture of potassium t-butoxide (9.0 g, 83.2 mmol) and 40 mL DMSO at 0°C, was added 5.0 g of (+)-11 base (10.4 mmol) and allowed to stir at room temperature. The orange reaction mixture was gently warmed to 45°C and after 30 min, GC analysis showed complete loss of starting material. After 2 h, 2.25 g potassium t-butoxide was added (20.8 mmol) and the reaction was completed in 15 min. The reaction mixture was cooled to 0°C, quenched with 100 mL H₂O and extracted with 3 x 100 mL CHCl₃. The organic phase was washed with 50 mL H₂O and evaporated to a yellow syrup. Addition and evaporation of MeOH gave crystalline product: 2.87 g (97%), mp 157-158°C, lit. Bartels-Keith, J. Chem. Soc. (C), 1966, 617-624, mp for (-)-12, mp 157-159°C. Anal. Calc. for CF₁₈H₁₉NO₃: C, 72.74; H, 6.39; N, 4.71. Found: C, 72.60; H, 6.48; N, 4.65. [α]_{D}²⁸ + 235.2 (C, 1.08, CHCl₃).

### Example 4

### (+)-14-Hydroxynorcodeinone, [(+)-13] (+)-

Northebaine [(+)-12] (14.8 g, 50 mmol) was added to a solution of 6.5 mL 88% formic acid and 26 mL 0.7% sulfuric acid, followed by addition of 7.2 mL 30% H₂O₂. The resulting heterogeneous reaction mixture became homogeneous and golden brown and was allowed to stir at room temperature for 48 h. Neutralization with 120 mL 10% Na₂CO₃ (pH 9.5) gave crystalline material that was filtered and washed with 50 mL H₂O and 100 mL MeOH to give 12.1 g (81%) cream-colored crystalline product. The aqueous filtrate was saturated with NaCl, extracted with 4 x 100 mL CHCl₃/MeOH (9:1). Evaporation of the extracts in vacuo and crystallization in MeOH resulted in 1.3 g (9%) crystalline product. Recrystallization of the combined material gave 13.4 ag (90%) product which was homogeneous by TLC; mp 209210°C. Anal. calc. for C₁₇H₁₇NO₄.3/4 H₂O: C, 65.29; H, 5.92; N, 4.47. Found: C, 65.12; H, 6.02; N, 4.45. [α]_{D}²⁵ + 171.73 (10.4, CHCl₃).

### Example 5

### (+)-Noroxycodone [(+)-18]

Catalytic hydrogenation of (-)-13 (4.5 g, 15 mmol) in 90 mL 10% glacial acetic acid (w/w) and 500 mg 5% Pd/BaSO₄ was followed by filtration over celite and washing the filter pad with 100 mL glacial acetic acid and 100 mL 10% glacial acetic acid. Neutralization of the filtrate with NH₄ OH to pH 9.5 and extraction with 3 x 50 mL CHCl₂ and 3 x 50 mL CHCl₃/MeOH 9:1 followed by removal of volatiles, in vacuo, resulted in a white powder 4.5 g (100% crude) that was 98.8% pure by GC analysis. Purification by preparation of the HI salt, in MeOH followed by recrystallization with MeOH/ether gave the HI salt of (+)-18 which was homogeneous by TLC. Anal. calc. for C₁₇H₁₉NO₄.HI : C, 47.58; H, 4.66; N, 3.26. Found: C, 47.55; H, 4.74; N,. 3.23.[α]_{D}²⁵ + 100.4 (C, 0.55, MeOH).

### Example 6

### (+)-Noroxymorphone [(+)-19]

O-Demethylation of (+)-18 was performed, dissolving 150 mg of (+)-18 (0.5 mmol) in 1.5 mL CHCl₃ and adding the solution to a stirring mixture of BBr₃ (0.3 mL, 30 mmol) in 1.5 mL CHCl₃ at 0°C. The pale yellow heterogeneous reaction mixture was allowed to stir at 0°C for 20 min and at room temperature for 40 min. The reaction was quenched by pouring onto 4 g ice/2 ml NH₄ OH (pH 9.5) and was allowed to stir at 0°C for 30 min. The white crystalline material was filtered, washed with 10 mL cold H₂O and 10 mL cold CHCl₃, and dried in a vacuum oven over night. The crystalline (+)-19, 80 mg (60% yield), was homogeneous and identical to authentic (-)-noroxymorphone by TLC, mp d >260°, mp (-)-noroxymorphone d >260°C, MS (CI) M+1 288, (HCl salt). The HCl salt of (+)-19 was formed in MeOH and gave (+)-19°HCl which was homogeneous by TLC, mp >260°C. [α]_{D}²² + 126.95 (C, 0.82, MeOH).

### Example 7

### (+)-Naloxone-3-0-methyl Ether.HCl [(+)-23.HCl]

A mixture of 2.1 g of (+)-18 (7 mmol), 21 mL dry DMF and 4.9 g anhydrous K₂CO₃ (35 mmol) was allowed to stir at 0°C. Allyl bromide (0.7 mL, 8 mmol) was added and the heterogeneous reaction mixture was allowed to stir at 0°C for 10 min and room temperature for 1.5 h. The inorganic material was removed by suction filtration and washed repeatedly with a total of 50 mL CHCl₃. The filtrate was then washed with 3 x 10 ml 10% Na₂CO₃ and 1 x 10 mL H₂O; evaporated in vacuo to a clear syrup, then dried on a vacuum pump at 50°C, resulting in 2.2 g white foam (92% crude). The crude product was dissolved in a minimum volume of hot 2-propanol and acidified in pH <2 with HCl/2 propanol. Crystallization and recrystallization in 2 propanol gave 2.8 g of (+)-23.HCl (86%) mp 248-250°C. Anal. calc. for C₂₀H₂₃NO₄.HCI.1/2 H₂O: C, 62.12; H, 6.21; N, 3.62. Found: C, 62.34; H, 6.49; N, 3.62. [α]²_{D}² + 176.11 (C, 0.95, MeOH).

### Example 8

### (+)-Naloxone [(+)-20]

Conversion of 680 mg of (+)-23.HCl (1.8 mmol) to the free base was performed in the usual manner. The dry, foamy free base was dissolved in 5 mL CHCl₃ and added dropwise to a stirring solution of 1.0 mL BBr₃ (10.7 mmol) in 5 mL CHCl₃ at 0°C. The addition funnel was washed with 2 ml CHCl₃ and the reaction mixture was allowed to stir at 0°C for 40 min then room temperature for 20 min. The reaction was quenched by pouring onto 15 g ice/4 mL NH₄ OH (pH 9) and allowed to vigorously stir at 0°C for 30 min. The organic layer was removed and the aqueous layer extracted with 4 x 10 mL CHCl₃. The organic fractions were combined and washed with 2 x 10 mL brine and 2 x 10 mL H₂O. Volatiles were removed in vacuo to give 450 mL white solid (80% crude). The crude product crystallized and was recrystallized in hot EtOAc to give 300 mg crystalline (+)-20 (60%), mp 167-168°. The free base was converted to the hydrochloride salt by dissolving in a minimum volume of 2 propanol and accordingly to pH 2 with HC1/2-propanol. Recrystallization in absolute ethanol gave (+)-20:HCl as white crystals, mp 206-210°C, lit. Merck Index, 10th ed., 1983 or (-)-2 0:HC 1, mp 200-205°C. Anal. Calc. C₁₉H₂₁NO₄:HCl:2H₂O: C, 57.10; H, 6.51; N, 3.50. Found: C, 57.07; H, 6.37; N, 3.37. [A]²_{D}² + 148.6 (0.97, MeOH).

### Example 9

### (+)-Naltrexone-3-0-methyl Ether.HCl.1 Isopropanol [(+)-24]

A heterogeneous solution of 5.57 g of (+)-18 (18.5 mmol), 50 mL dry DMF and 13.0 g anhydrous K₂CO₃ (92.5 mmol) was allowed to stir at 0°C. Bromomethylcyclopropane (2.2 mL, 22 mmol) was added and the heterogeneous reaction mixture was allowed to stir at 0°C for 10 min, then for 24 h at room temperature. The inorganic material was removed by suction filtration and washed with 200 mL CHCl₃. The filtrate was washed with 3 x 100 mL 10% aqueous Na₂CO₃ and 100 mL H₂O and the volatiles were removed in vacuo. Distillation of toluene and drying at 50°C in high vacuum gave a white foamy product, 5.9 g (90% crude). The free base was converted into the hydrochloride salt by dissolving in a minimum volume of hot 2-propanol and acidifying to pH 2 with HC1/2-propanol. Crystallization and recrystallization gave 7.0 g (84%) of (+)-24.HCl.1 isopropanol white crystalline salt; mp 235-238°C. Anal. Calc. C₂₁H₂₅NO₄:HCl:3/2 H₂O: C, 60.23; H, 6.93; N, 3.34. Found: C, 60.41; H, 6.89; N, 3.34.

### Example 10

### (+)-Naltrexone [(+)-21]

Conversion of 2.35 g (+)-24.HCl isopropanol into the free base was performed by extraction from 10% aqueous Na₂CO₃ into CHCl₃ to give 2.1 g of white foamy free base which was dissolved in 18 mL CHCl₃ (dried over 3A molecular sieves) and added dropwise to a stirring solution of 3.5 mL BBr₃ (freshly distilled over Hg) and 18 mL CHCl₃ at 0°C. The addition funnel was washed with 7 mL CHCl₃ and the reaction was allowed to stir for 20 min at 0°C and 45 min at room temperature. The reaction mixture was quenched on 53 g ice/18 mL NH₄ OH (pH 9.5) and after washing the reaction flask with aqueous NH₄OH/CHCl₃, it was allowed to stir for 30 min at 0°C. The aqueous mixture was extracted with 4 x 30 mL CHCl₃ and the organic phase was washed with 2 x 30 mL H₂O and 2 x 50 mL brine, dried (Na₂SO₄) and the volatiles were removed in vacuo resulting in 1.8 g white foam (88% crude). Crystallization and recrystallization in EtOAc gave 1.15 g of (+)-21 white crystals (57%); mp 155°C. Anal. calc for: C₂₀H₂₃NO₄.1/4 EtOAc: C, 69.44; H, 6.88; N, 3.85. Found: C, 69.42; H, 6.88; N, 4.00.[α]²_{D}² + 194.51 ;(C, 0.82, MeOH).

### Example 11

### (+)-Nalmefene.HCl [(+)-22.HCl]

This compound can be prepared from (+)-naltrexone [(+)-21] as described by Hahn, et al, J. Med. Chem., 1975, Vol. 18, pp 259-262, for the (-)-isomer. The (+)-compound showed mp 186-187°C, (-)-nalmefene, mp 187-189°C (GC analysis showed 100% purity, RT = 7.42, 220°C). The hydrochloride salt was prepared in 2-propanol with HCl/2-propanol (pH 2), and crystallized and recrystallized in 2 propanol to give (+)-nalmefene.HCl mp 199-200°C, lit. Hahn, et al, J. Med. Chem., 1975, Vol. 18, pp 259-262, mp for (-)-nalmefene.HCl 197-198°C. Anal. calc. for C₂₁H₂₅NO₃.HCl.1/2 H₂O: C, 65.47; H, 7.41; N, 3.45. Found: C, 65.52; H, 7.07; N, 3.63. [c]²² + 1.43.7 (C, 1.05, MeOH), corrected for anhydrous + 147.2 [α]_{D}²² (authentic (-)-145.4 (C, 1.04, MeOH).

### Example 12

### (+)-Norcodeinone Dimethyl Ketal [(+)-37]

Conversion of 2.5 g of (+)-11 to the free base (2.13 g foam, 4.26 mmol) was done with 20% NH₄ OH and ether extraction followed by drying. To a solution of the free base in 42 mL dry THF, 1.15 g potassium t-butoxide (8.5 mmol) was added and the reaction mixture was allowed to stir at room temperature, under an atmosphere of argon for 24 h. GC analysis showed the reaction was complete (RT = 5.17, 220°C); the THF was removed in vacuo and 15 ml H₂O was added. The (+)-37 was isolated as a white feathery dihydrate (as seen in NMR analysis) 1.60 g (100%) mp 113-114°C, lit., Bartels-Keith, J. Chem. Soc. (C), 1966, 617-624, for (-)-37, mp 113-114°C. Anal. calc. for C₁₉H₂₃NO₄.3/4 H₂O: C, 66.58; H, 7.14; N, 4.08. Found: C, 66.49; H, 7.23; N, 4.00 _{D}²⁸ + 215.9 (C, 1.09, CHCl₃).

### Example 13

### (+)-Norcodeine [(+)-6]

A solution of 1.0 g (+)-37 (3 mmol) in 15 mL 3N HCOOH (2.35 g 88% HCOOH in a total volume of 15 mL H₂O) was allowed to stir at room temperature for 20 min at which time GC analysis showed complete loss of starting material. The reaction mixture was neutralized to pH 5.5-6.0 with portionwise addition of solid NaHCO₃ at 0°C¹. Sodium borohydride (90 mg, 3.4 mmol) was added and the reaction mixture, was allowed to stir for 20 min. To the completed reaction mixture, 6 ML 1.0 N NaOH was added and extraction with 3 x 10 mL CHCl₃ followed by removal of solvent in vacuo resulted in a white foam which crystallized in 2-propanol to give a(+)-6. 750 mg (90%) mp 178-181°C, d-authentic sample of (-)-6, mp 181-183°C. Anal. Calc. for C₁₇H₁₉NO3: C, 71.60; H, 6.66; N, 4.91. Found: C, 71.45; H, 6.76; N, 4.84. [α]²_{D}⁸ + 115.23 (C, 1.09, CHCl₃).
¹Note the intermediate nocodeinone [(+)-38] can be isolated as a white foam by solvent extraction and evaporation.

### Example 14

### (+)-Nalorphine-O-methylether [(+)-40]

To a solution of 540 mg (+)-norcodeine [(+)-39] (1.9 mmol) in 6 mL of dry DMF, 1.33 g K₂CO₃ (9.5 mmol) and 0.19 mL allyl bromide (2.1 mmol) were added at 0°C. The reaction mixture was allowed to stir at 0°C for 10 min, then at room temperature for 50 min. Inorganic material was removed by suction filtration and washed with 10 mL CHCl₃. Volatiles were removed in vacuo from the filtrate which was then extracted with 3 x 10 mL CHCl₃ from 10 mL 10% Na₂CO₃ followed by washing of the organic phase with 10 mL H₂O and evaporation of solvent. Crystallization from ether gave 445 mg white crystalline (+)-40 (72%) that was homogeneous by TLC. mp 91-93°C, lit, J. Am. Chem. Soc., 1942, 64:869, mp for (-)-40, 93°C. Anal. Calc. for C₂₀H₂₃NO₃: C, 73.86; H, 7.07; N, 4.31. Found: C, 73.76; H, 7.13; N, 4.28. [α]²_{D}⁵ + 130.40 (C, 0.99, CHCl₃).

### Example 15

### (+)-Nalorphine [(+)-41]

A solution of 235 mg (+)-40 (.72 mmol) in 1.5 mL CHCl₃ was added dropwise over 1 min to a solution of 0.44 mL BBr₃ (4.3 mmol freshly distilled over Hg) in 13 mL CHCl₃ at 0°C. The addition funnel was washed with 1 mL CHCl₃ and the reaction mixture was allowed to stir at 0°C for 20 min and room temperature for 20 min. The yellow heterogeneous reaction mixture was poured onto 6 g ice/3 mL NH₄ OH (pH 9.5) and allowed to stir at 0°C for 45 min. The volatiles were removed in vacuo resulting in a gummy product which was dissolved in 5 mL CHCl₃ and washed with 2 x 10 mL 10% NH₄ OH; the aqueous phase was then washed with 5 x 10 mL CHCl₃ and the combined CHCl₃ fractions were washed with 1 x 10 mL H₂O. Evaporation of solvent and distillation of toluene resulted in 300 mg crude product which crystallized in ether, to give 200 mg of (+)-41, (89%) mp 188-191°C, lit, Merck Index, 10th ed., 1983, mp for (1)-41, 208-209°C. Purification by formation of the hydrochloride salt in 2-propanol and recrystallization in MeOH gave 150 mg (45%) of pure HCl (+)-41 as the salt, mp d >260, lit. Merck Index, 10th ed., 1983, mp for (-)-41:HCl d 269°C. [α]²_{D}⁵ + 99.91 :(C, 1.06, MeOH).

It is understood that in the claim structure below the compounds in their preparation and use are made both in the natural (generally (-)-) and the unnatural (generally (+)-) stereochemical series.

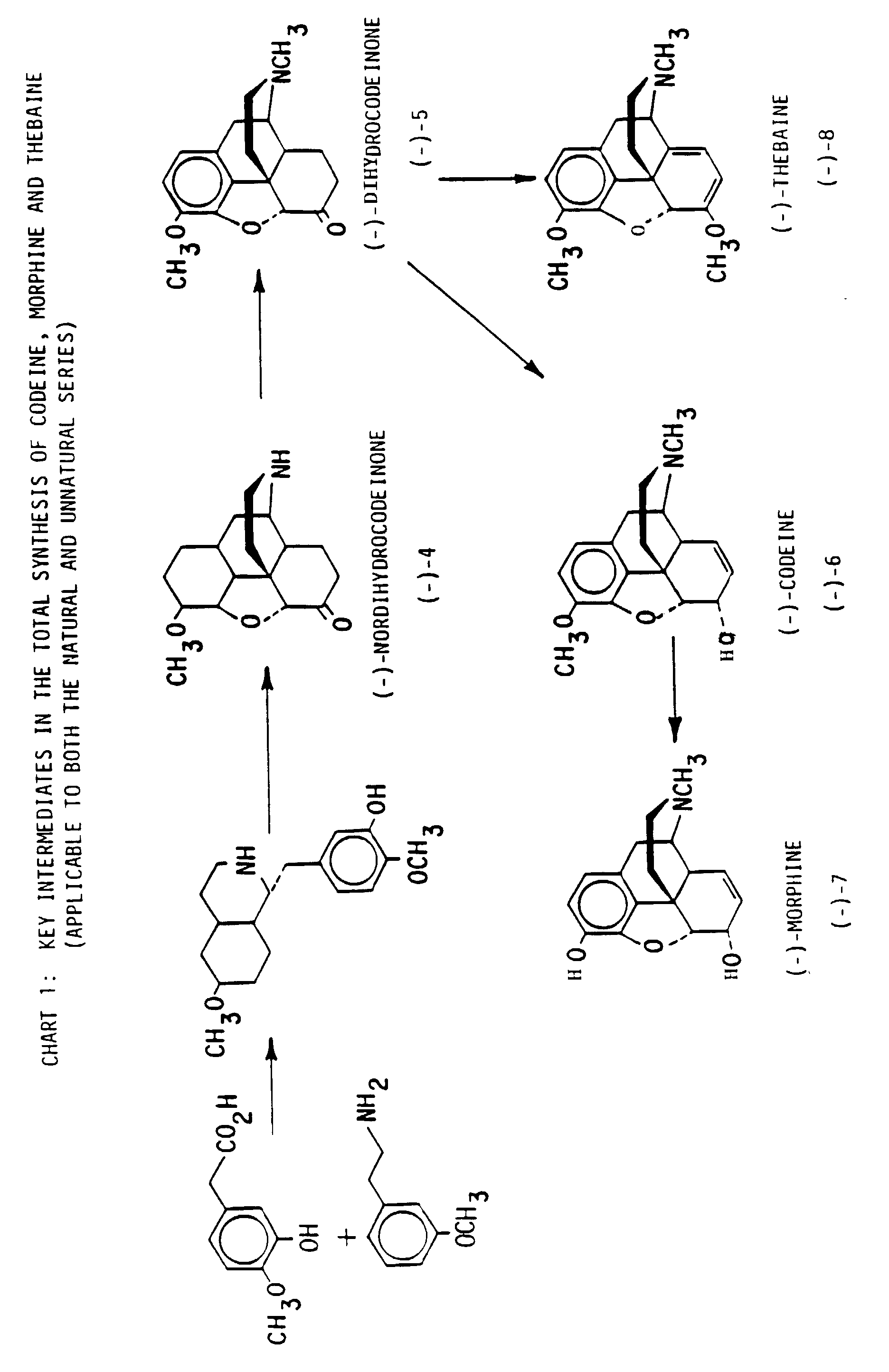

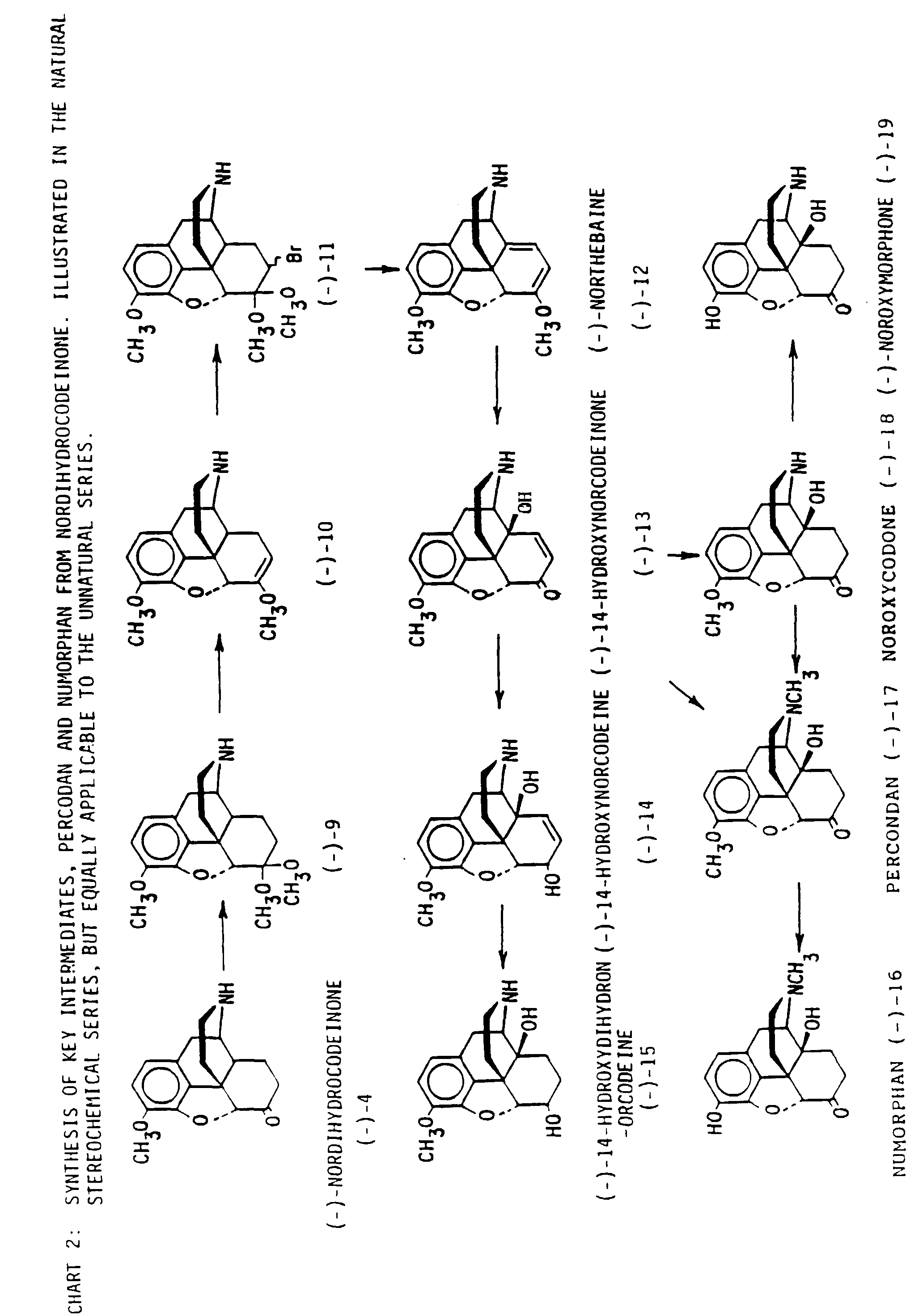

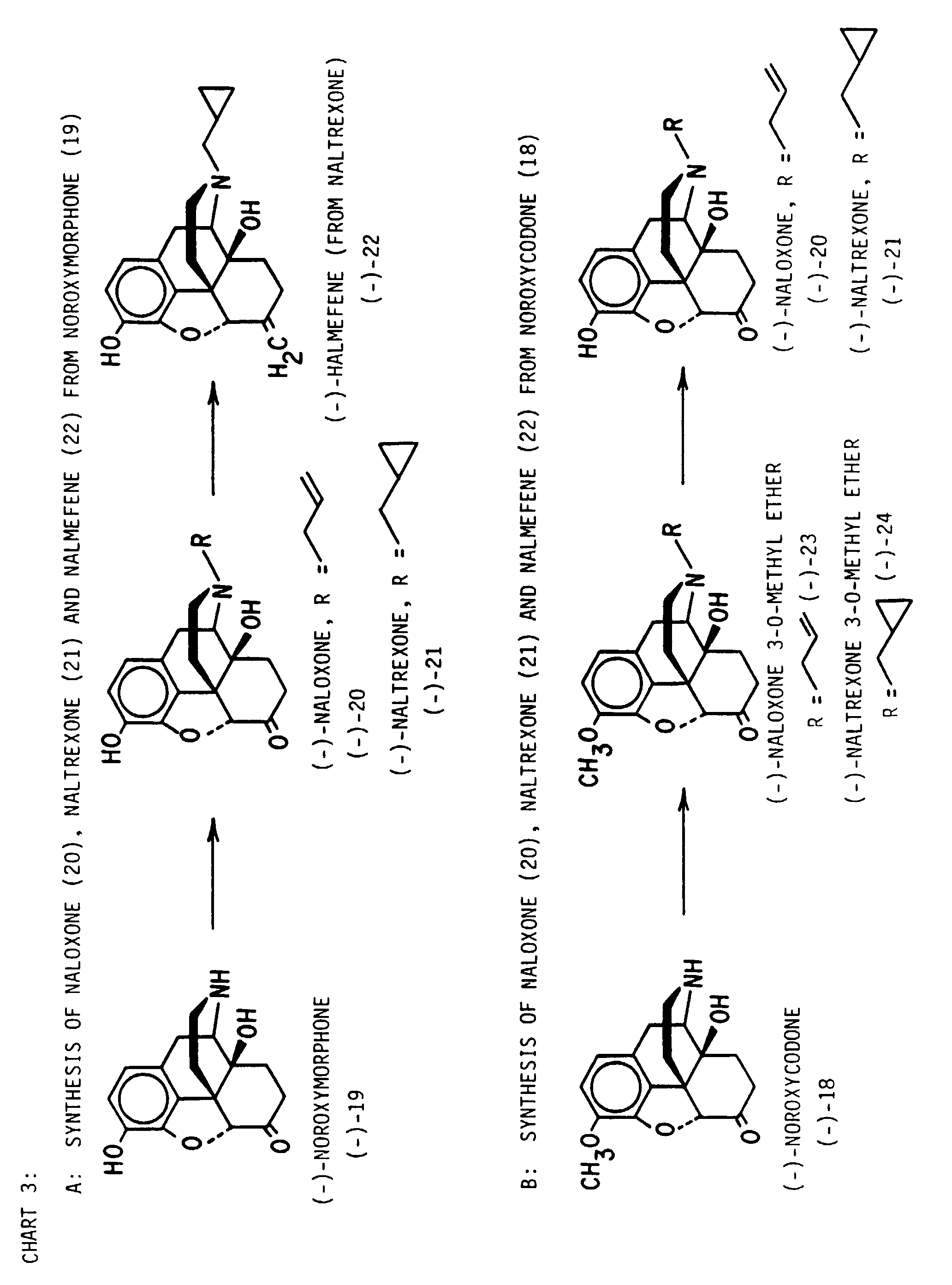

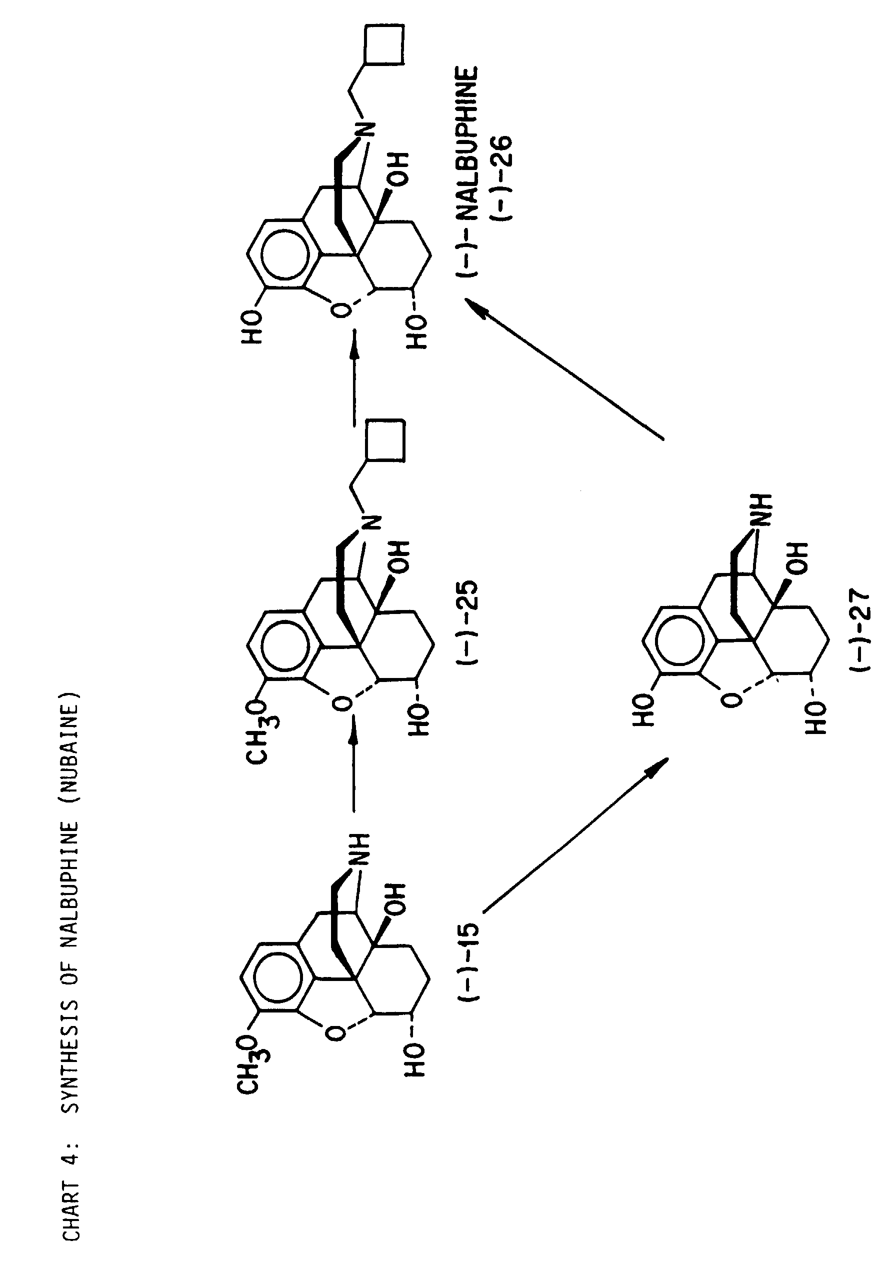

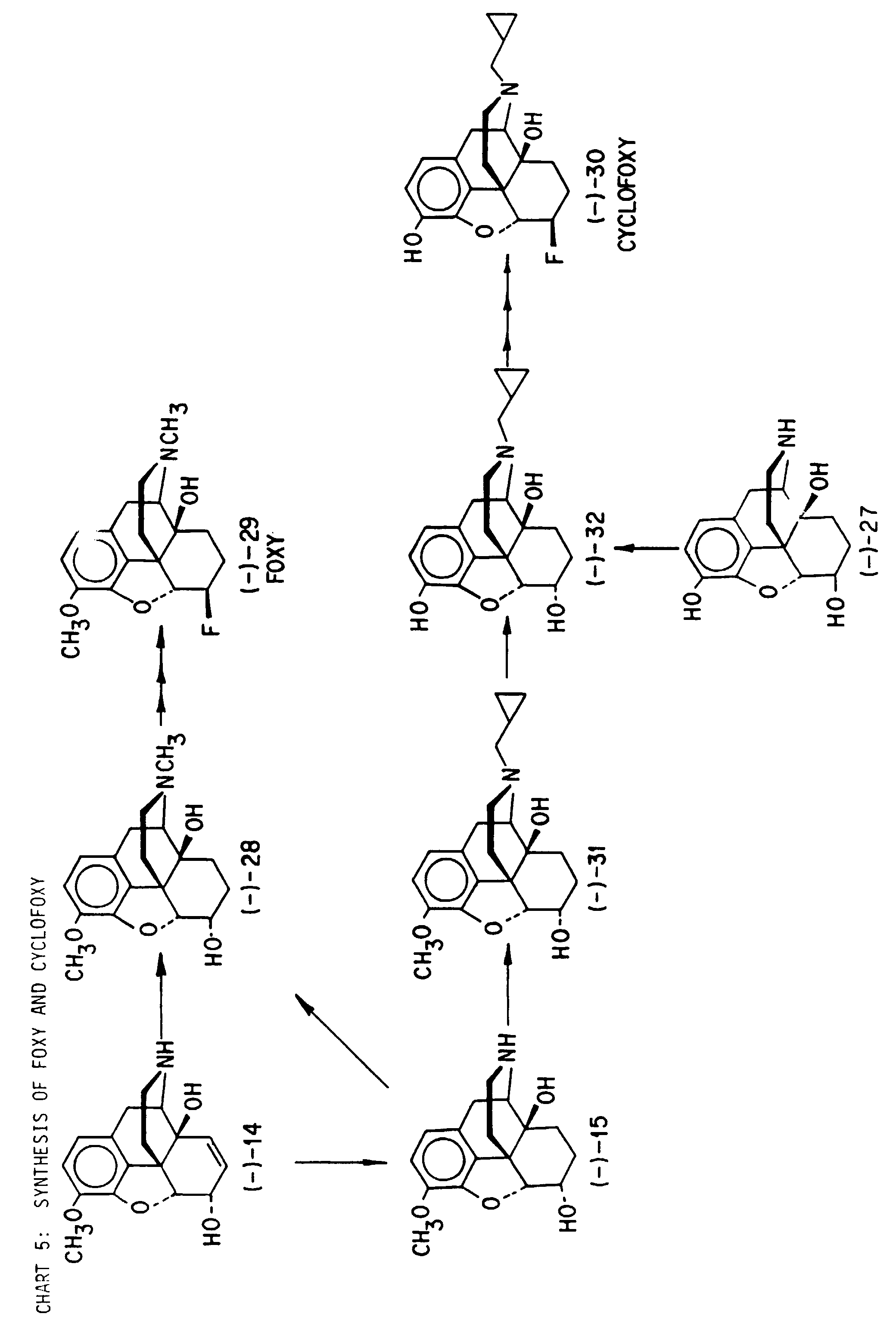

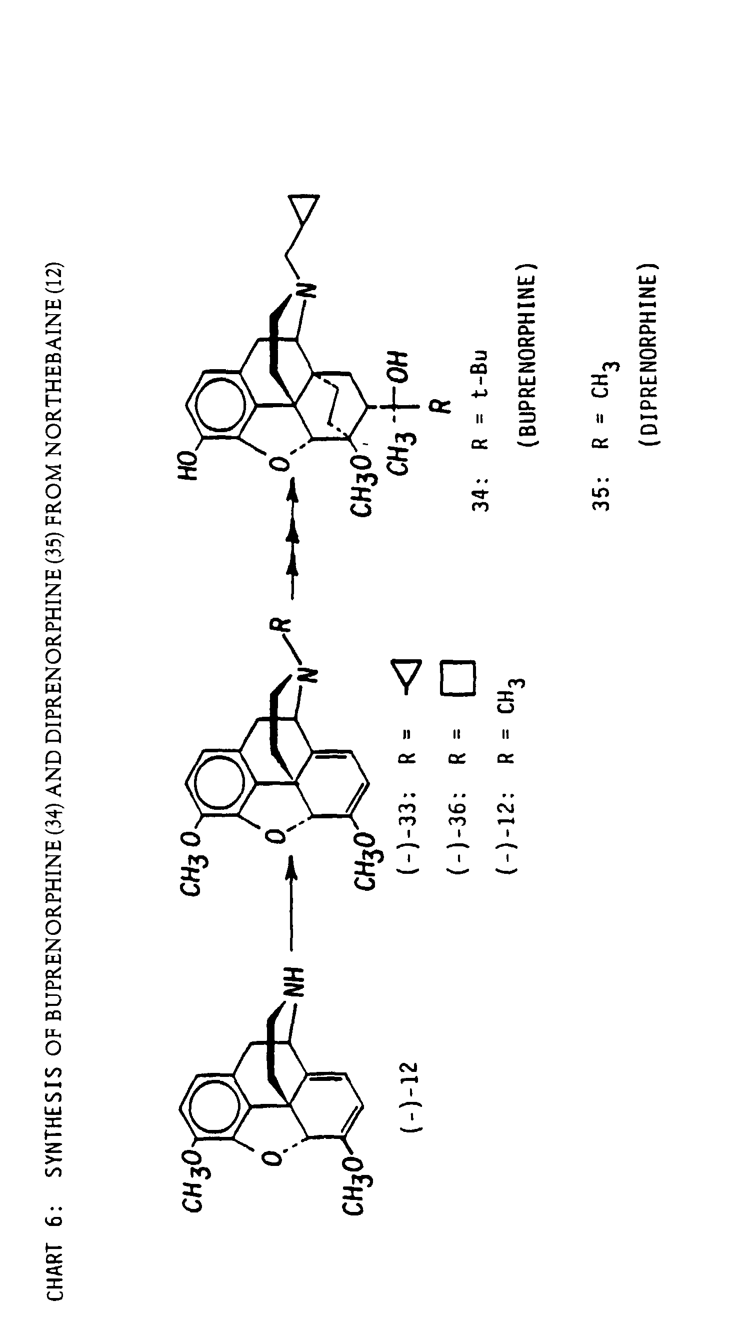

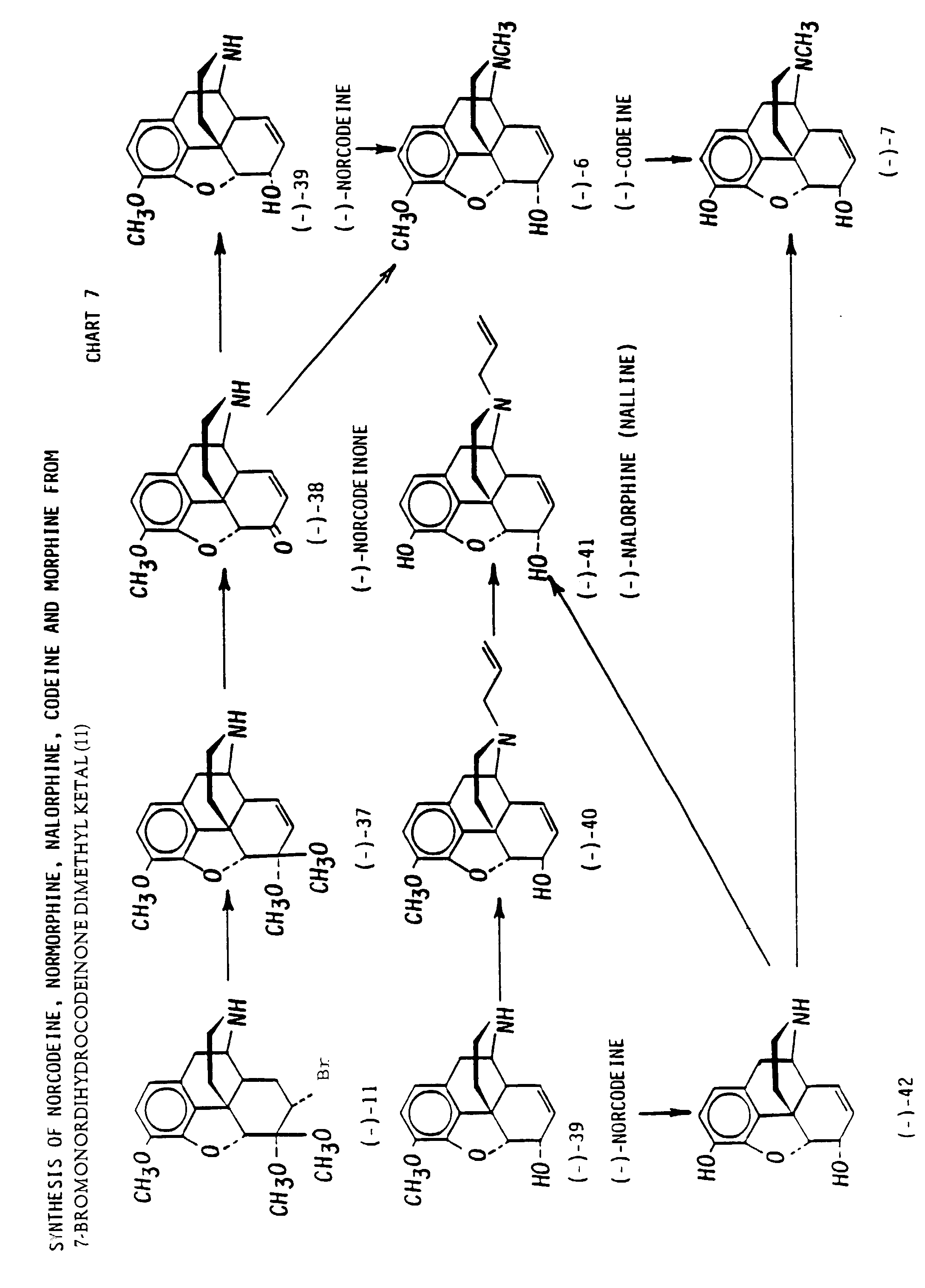

## Claims

1. A process of producing an intermediate for the production of opiate narcotics, agonist-antagonist drugs, and stereoisomers thereof, comprising the steps of:
(a) refluxing nordihydrocodeinone having an unsubstituted N-substituent with an organic acid and trialkylorthoformate in a short chain alcohol ROH, where R is C₁-C₄ alkyl under an inert atmosphere to yield an alcoholic mixture of dialkyl ketal (I): 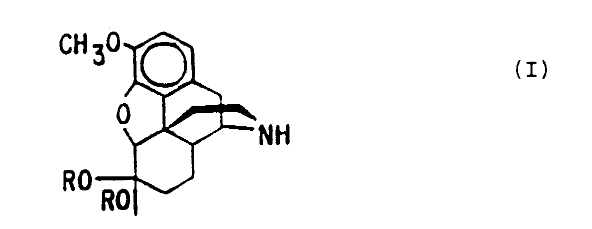 and a delta-6-enol ether (II): 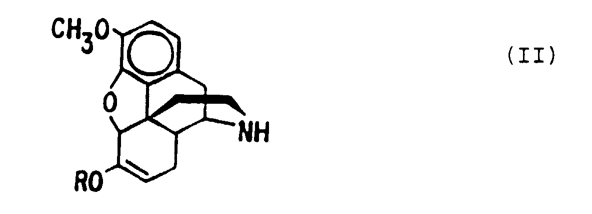
(b) completely converting the dialkyl ketal (I) in said mixture to the delta-6-enol ether (II);
(c) reacting the delta-6-enol ether (II) from step (b) with an organic acid and a halogenating agent to yield a hydrohalide salt of a 7-halo-dialkyl ketal (III) : 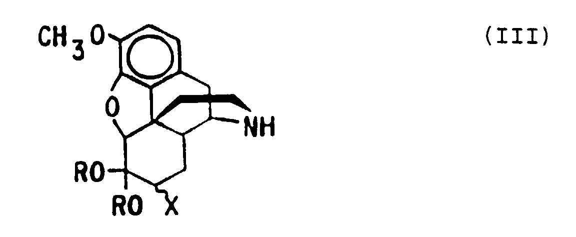 where X=halo, wherein said process occurs without substitution of said N-substituent.

2. A process as claimed in claim 1, wherein step (b) is performed by continuously adding a dry aprotic solvent to said mixture and removing said alcoholic solvent, and alcohol formed during said conversion, by distillation.

3. A process as claimed in claim 1, wherein step (b) is performed by addition of a base.

4. A process as claimed in claim 3, wherein said base is organic.

5. A process as claimed in claim 4, wherein said base is potassium t-butoxide.

6. A process as claimed in any one of the preceding claims, further comprising dehalogenating said 7-halodialkyl ketal (III) by reaction with a base in an aprotic solvent to produce northebaine.

7. A process as claimed in claim 6, further comprising oxidizing said northebaine to yield 14-hydroxynorcodeinone.

8. A process as claimed in claim 7, wherein said oxidation of northebaine to 14-hydroxynorcodeinone is accomplished by reaction with a performic acid.

9. A process as claimed in any one of claims 1-5, further comprising dehalogenating said 7-halodialkylether by reaction with a base in an ether or hydrocarbon solvent to yield norcodeinone ketal.

10. A process as claimed in claim 9, wherein said solvent is tetrahydrofuran and said base is potassium t-butoxide.

11. A process as claimed in claims 9 or 10, further comprising hydrolyzing said norcodeinone ketal to norcodeinone, and reducing said norcodeinone to yield norcodeine.

12. A process as claimed in claim 11, wherein said reducing is by reaction with an alkali butoxide.

13. Hydrobromide salt of optically active crystalline (+)7-bromodialkyl ketal of nordihydrocodeinone (III) and salts thereof.

## Patentansprüche

1. Verfahren zur Herstellung eines Zwischenproduktes zur Herstellung von Opiat-Narkosemitteln, Agonist-Antagonist-Medikamenten und Stereoisomeren davon, mit folgenden Schritten:
(a) Rückströmen von Nordihydrocodeinon mit einem unsubstituierten N-Substituenten mit einer organischen Säure und Trialkylorthoformat in einen kurzkettigen Alkohol ROH, wobei R ein C₁-C₄ Alkyl unter einer inerten Atmosphäre ist, um eine alkoholische Mischung aus Dialkyl-ketal (I): 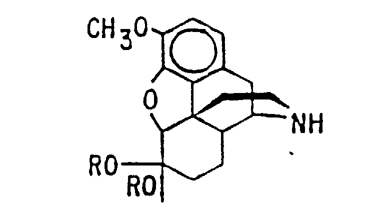 und einen Delta-6-Enol Ether (II) zu gewinnen: 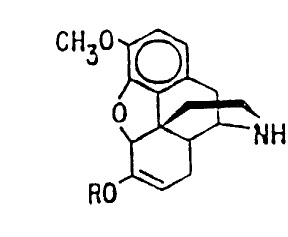
(b) vollständiges Umwandeln des Dialkyl-ketal (I) in der Mischung zu Delta-6-Enol Ether (II);
(c) Reagieren des Delta-6-Enol Ether (II) aus Schritt (b) mit einer organischen Säure und einem halogenierten Mittel, um ein Hydrohalid-Salz eines 7-Halo-Dialkyl Ketal (III) zu gewinnen: 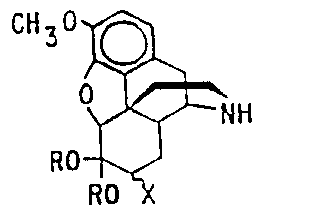 wobei X=Halo und wobei das Verfahren ohne Substitution des N-Substituenten abläuft.

2. Verfahren nach Anspruch 1, bei dem Schritt (b) durch kontinuierlichen Zusatz eines trockenen aprotischen Lösungsmittels zu der Mischung und Entfernen des alkoholischen Lösungsmittels und des während der Umwandlung gebildeten Alkohols durch Destillation durchgeführt wird.

3. Verfahren nach Anspruch 1, bei dem Schritt (b) durch Zusatz einer Base durchgeführt wird.

4. Verfahren nach Anspruch 3, bei dem die Base organisch ist.

5. Verfahren nach Anspruch 4, bei dem die Base Kalium t-butoxid ist.

6. Verfahren nach einem der vorhergehenden Ansprüche, mit einer Dehalogenierung des 7-Halodialkyl ketal (III) durch Reaktion mit einer Base in einem aprotischen Lösungsmittel zur Erzeugung von Northebain.

7. Verfahren nach Anspruch 6, mit einer Oxidierung des Northebain, um 14-Hydroxynorcodeinon zu gewinnen.

8. Verfahren nach Anspruch 7, bei dem die Oxidierung von Northebain zu 14-Hydroxynorcodeinon durch Reaktion mit einer Perameisensäure erreicht wird.

9. Verfahren nach einem der Ansprüche 1 bis 5, mit einer Dehalogenierung des 7-Halodialkylether durch Reaktion mit einer Base in einem Ether oder Hydrocarbon-Lösungsmittel, um Norcodeinone ketal zu gewinnen.

10. Verfahren nach Anspruch 9, bei dem das Lösungsmittel Tetrahydrofuran und die Base Kalium T-butoxid ist.

11. Verfahren nach Anspruch 9 oder 10, mit einer Hydrolyse des Norcodeinon ketal zu Norcodeinon und einer Reduktion des Norcodeinon, um Norcodeine zu gewinnen.

12. Verfahren nach Anspruch 11, bei dem die Reduktion durch Reaktion mit einem Alkali butoxid erfolgt.

13. Hydrobromid-Salz mit optisch aktivem kristallinen (+)7-Bromodialkyl ketal von Nordihydrocodeinon (III) und Salze davon.

## Revendications

1. Procédé de production d'un intermédiaire de fabrication de narcotiques opiacés, de médicaments agonistes-antagonistes, et de stéréoisomères de ceux-ci, consistant en les étapes de :
(a) porter au reflux de la nordihydrocodéinone ayant un N-substituant non substitué avec un acide organique et un trialkylorthoformate sur un alcool ROH à chaîne courte, dans lequel R représente un groupe alkyle en C₁ à C₄ sous une atmosphère non réactive pour obtenir un mélange alcoolique de dialkyl-cétal (I) : 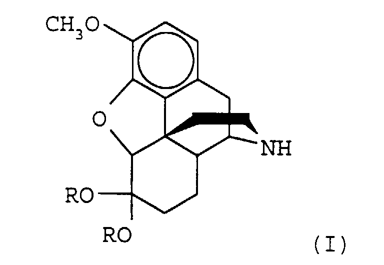 et un delta-6-énol éther (II) : 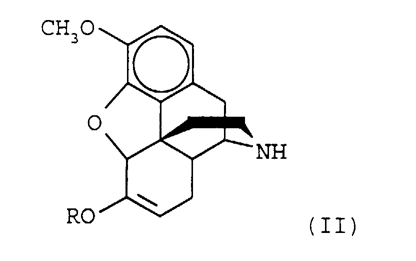
(b) transformer complètement le dialkyl-cétal (I) dans ledit mélange en le delta-6-énol éther (II);
(c) faire réagir le delta-6-énol éther (II) de l'étape (b) avec un acide organique et un agent halogénant pour obtenir un sel d'hydrohalogénure d'un 7-halo-dialkyl-cétal (III) : 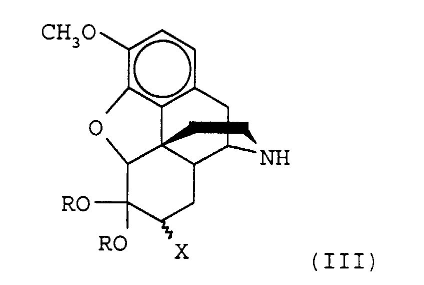 dans lequel X = halogène, dans lequel ledit procédé a lieu sans substitution dudit N-substituant.

2. Procédé selon la revendication 1, dans lequel l'étape (b) est effectuée en ajoutant en continu un solvant anhydre aprotique audit mélange et en éliminant ledit solvant alcoolique, et l'alcool formé durant ladite transformation, par distillation.

3. Procédé selon la revendication 1, dans lequel l'étape (b) est effectuée par ajout d'une base.

4. Procédé selon la revendication 3, dans lequel ladite base est organique.

5. Procédé selon la revendication 4, dans lequel ladite base est du t-butylate de potassium.

6. Procédé selon l'une quelconque des revendications précédentes, comprenant en outre la déshalogénation dudit 7-halodialkyl-cétal (III) par réaction avec une base dans un solvant aprotique pour obtenir de la northébaïne.

7. Procédé selon la revendication 6, comprenant en outre l'oxydation de ladite northébaïne pour obtenir la 14-hydroxynorcodéinone.

8. Procédé selon la revendication 7, dans lequel ladite oxydation de la northébaine en 14-hydroxynorcodéinone est effectuée par réaction avec un acide performique.

9. Procédé selon l'une quelconque des revendications 1-5, comprenant en outre la déshalogénation dudit 7-halodialkyléther par réaction avec une base dans un solvant éther ou hydrocarbure pour donner la norcodéinone-cétal.

10. Procédé selon la revendication 9, dans lequel ledit solvant est le tétrahydrofuranne et ladite base est le t-butylate de potassium.

11. Procédé selon les revendications 9 ou 10, comprenant en outre l'hydrolyse de ladite norcodéinone-cétal en norcodéinone, et la réduction de ladite norcodéinone pour obtenir la norcodéine.

12. Procédé selon la revendication 11, dans lequel ladite réduction se fait par réaction avec un butylate alcalin.

13. Sel hydrobromure du (+)7-bromodialkyl-cétal optiquement actif cristallisé de la nordihydrocodéinone (III) et les sels de celle-ci.
